# EUROPEAN PATENT SPECIFICATION

(11) **EP 3 612 116 B1**
(45) Date of publication and mention of the grant of the patent: **16.09.2020**
(21) Application number: 18717934.6
(22) Date of filing: 18.04.2018
(51) Int. Cl.: A61B 17/74

(54) **LONGITUDINAL BONE IMPLANT**
LÄNGLICHES KNOCHENIMPLANTAT
IMPLANT OSSEUX LONGITUDINAL

(30) Priority: 20.04.2017 EP 17167216
(43) Date of publication of application: 26.02.2020
(73) Proprietor: Stöckli Group AG, 6214 Schenkon (CH)
(72) Inventor: WELTE, Thomas, 79576 Weil am Rhein (DE); SCHWER, Stefan, 79540 Lörrach (DE); KAEWMULPET, Christopher, Danai, 4600 Olten (CH); GRAF, GOITIA, ORTIZ, Maria, Elisa, 3008 Bern (CH)
(74) Representative: Banse & Steglich Patentanwälte PartmbB
(86) International application number: PCT/EP2018/059900
(87) International publication number: WO 2018/192970

(56) References cited:
- DE-U1-202004 018 748
- US-A1- 2001 007 074
- US-A1- 2013 253 594
- US-A1- 2016 081 810

## Description

The invention relates to a longitudinal bone implant with a substantially circular cross-sectional profile, comprising a front section having a front end and a shaft section having a rear end, wherein the front section comprises at least three longitudinal groove-like cut-outs extending in the axial direction of the front section and opening towards the front end of the implant, circumferentially alternating with at least three longitudinal, radially protruding ribs extending in an axial direction, wherein the ribs have an increased cross-sectional width in the section radially more distant to the central longitudinal axis of the implant as compared to the width in a section radially closer to the central longitudinal axis of the implant.

### State of the art

Fractures in the proximal section of long bones, especially neck or head fractures of the femur or the humerus, are generally treated by implanting medical devices to stabilize the fracture. The fixation of femur fractures is especially demanding due to the high mechanical stress imposed on the femur neck. The aforementioned fractures are commonly stabilized by a combination of two bone implants. The first implant can be an intramedullary nail comprising a transversal bore which is implanted into the intramedullary channel of the proximal section of the bone. Alternatively, an angular plate, comprising a bone plate that is attached to the outside of the proximal section of the bone and a sleeve angularly attached to the plate, may be used. The broken head or neck is fixed to the proximal section of the bone by a second implant, which is a cross nail, a cross screw or a blade, which is implanted into the head or neck of the femur or humerus and assembled into the transversal bore of the intramedullary nail or the sleeve of the angular plate. To provide for a proper long-term fixation, the cross nail, screw or blade has to be stably implanted into the bone to prevent a movement of said device in the bone. Furthermore, upon final implantation, a tight connection between the intramedullary nail or angular plate and the cross nail, screw or blade should prevent a rotation or longitudinal movement of the cross nail, screw or blade.

A simple cross nail is disclosed in U.S. 3,433,220. The nail comprises a proximal part with longitudinal ribs and grooves for engagement with the bone and a distal part with circumferential grooves and ribs for engagement with a setting screw in an intramedullary nail. However, the structure of the proximal part may allow a backing out of the nail from the bone due to limited interaction of the nail with the spongiosa. Furthermore, the interaction of the distal part and the cross nail with the intramedullary nail may inhibit, but not prevent a rotation of the cross-nail in the transversal bore of the intramedullary nail.

EP 2 330 993 B1 discloses a screw which is inserted through an intramedullary nail into the femur head. The screw has a threaded proximal part for interaction with the femur head and a distal shaft section, comprising longitudinal grooves, for insertion into an intramedullary nail. The front part has a relatively great diameter and therefore results in a substantial displacement of spongiosa. Furthermore, the engagement of the thread with the bone is limited due to the relatively small height of the teeth of the thread. Thus, the proximal part of the screw may not prevent rotation of the device in the relatively soft spongiosa and is frequently not able to prevent a rotational dislocation of the femur head.

The implant disclosed in EP 1 233 712 B1 tries to address the problem of rotational instabilities of screws by inserting a second screw into the femur head through a second transversal bore in the intramedullary nail. However, implanting a second screw into the femur head increases the complexity of the surgical procedure. Furthermore, only intramedullary nails comprising a second transversal bore can be used in this procedure.

As an alternative to threaded screws, EP 0 961 587 B1, EP 1 435 862 B1 and EP 2 018 127 B1, for example, disclose devices for implementation into the femur head having a proximal section configured with a plurality of helically twisted blades. The distal part of the devices is either configured for insertion into an intramedullary nail or into the sleeve of an angled bone plate. While the blades provide an increased surface for the interaction with the spongiosa, a lateral movement of the fractured femur head along the axis of the device induces a rotation of the fracture through the helical twist of the blades. Thus, the stabilization of the fracture is impaired.

An alternative design for a device comprising blades is disclosed in EP 0 701 419 B1. The front part of the device comprises two substantially T-formed ribs. The cross-sectional profile of the device is not circular, but comprises two opposite flat sides and two opposing narrow convex sides, to prevent a rotation of the device in a correspondingly shaped transversal bore of the intramedullary nail. However, due to the non-circular cross-sectional profile of the device, the device cannot be used with intramedullary nails having a circular transversal bore. Upon implantation into the femur head, one of the narrower convex sides of the implants is directed in a cranial direction. Accordingly, while the highest mechanical load is imposed on the implant from cranial direction, the surface for absorbing said load is reduced in this direction. Furthermore, the orientation of one of the T-formed ribs in the cranial direction destroys the spongiosa in the direction of the highest mechanical stress. However, it is desirable to maintain the spongiosa in the cranial direction to improve the stability of the implant in the bone.

Document US2001/007074 A1 discloses a longitudinal bone implant according to the preamble of claim 1.

In summary, the prior art devices exhibit different design features that result in an impaired stability, especially rotational stability, of the implants in the bone.

### Problem underlying the invention

In view of the prior art, it was the general problem underlying the present invention to provide bone implants, uses and methods which overcome the above-mentioned disadvantages of the prior art. Especially, the implants should have a good stability in the bone and should be suitable for absorbing high mechanical loads. It is also desirable to preserve spongiosa during the insertion of the implant into the bone, especially in the direction from which the highest mechanical loads are imposed on the implant. Furthermore, the implants should be suitable for use with common intramedullary nails having a circular transversal bore.

### Disclosure of the invention

Surprisingly, it was found that the problem underlying the invention is overcome by the bone implant and its uses according to the claims. Further embodiments of the invention are outlined throughout the description.

Subject of the invention is a longitudinal bone implant with a substantially circular cross-sectional profile, comprising a front section having a front end and a shaft section having a rear end, wherein the front section comprises at least three longitudinal groove-like cut-outs extending in the axial direction of the front section and opening towards the front end of the implant, circumferentially alternating with at least three longitudinal, radially protruding ribs extending in an axial direction, wherein the ribs have an increased cross-sectional width in a section radially more distant to the central longitudinal axis of the implant as compared to the width in a section radially closer to the central longitudinal axis of the implant.

In a highly preferred embodiment the front section is non-threaded.

The bone implant according to the present invention is a device suitable for complete or partial insertion into a bone, preferably a long bone, more preferably into the humerus or the femur. Most preferably, the device is suitable for insertion into the femur, preferably the femur neck and/or head.

The bone implant according to the present invention has a substantially cross-sectional profile. A "cross-sectional profile", within the meaning of the present invention, is the outline of the projection of the whole body of the implant on the plane vertical to the longitudinal axis of the implant. Within the meaning of the present invention, "cross-sectional" or the like always refers to a section in a plane vertical to the longitudinal axis of the implant, whereas "longitudinal cross-section" or the like refers to a section in the plane of the longitudinal axis of the implant. Within the meaning of the present invention, a "substantially circular" profile is a profile that significantly matches a circle with a given radius. In a "substantially circular" profile, the outline may have protrusions or indentions in comparison to a circle. Preferably the outline may have indentions. In specific embodiments, at least 60%, at least 70%, preferably at least 80% and/or more preferably at least 90% of the profile may match a circle with a given radius. Most preferably, the profile may be circular.

The implant comprises different sections along its longitudinal axis. The implant has a front section, having a front end and a shaft section having a rear end. The implant may have several sections between the front section and the shaft section. When implanted into the bone of a subject, the front section may be directed in a proximal direction relatively to the center of the body of the subject and the shaft section may be directed into distal direction. Thus, the terms front/proximal and rear/distal are used interchangeably within the context of the present invention, where technically sensible.

According to the present invention, the implant has groove-like cut-outs extending in an axial direction. Within the meaning of the present invention, a "groove-like cut-out" refers to a longitudinally extending indention in the body of the implant, irrespective of whether these indentions were obtained by cutting out material from a body or by any other means of manufacture. The cut-outs open towards the front and of the implant. Thus, the volume of the cut-out is not confined by material in a lateral direction towards the front end of the implant.

The ribs and groove-like cut-outs alternate circumferentially relative to the central longitudinal axis of the implant. Thus, the ribs and grooves alternate clockwise/counterclockwise with regard to the central longitudinal axis of the implant.

The term "radially protruding ribs" refers to ribs which in the cross-section of the front section of the implant protrude over the grooves in a radial direction relative to the central longitudinal axis of the implant. The ribs furthermore extend in a longitudinal axial direction. Thus, the longest extension of the ribs is in the longitudinal direction of the device.

The ribs have an increased cross-sectional width in a section radially more distant to the central longitudinal axis. Within the meaning of the present invention, the "cross-sectional" width of the rib refers to the width of the rib in the plane vertical to the longitudinal axis of the implant. At least, the width of a section of at least one rib radially more distant to the longitudinal axis is greater as compared to the width of a second section, radially closer to the longitudinal axis of the implant. This definition, within the meaning of the present invention, encompasses ribs which are cross-sectionally substantially T-formed, wherein the base of the "T" is directed towards the longitudinal axis of the implant, or which are cross-sectionally substantially L-formed, wherein the top of the "L" is directed towards the longitudinal axis of the implant. T-formed ribs are a highly preferred embodiment of the invention. Furthermore, the definition encompasses embodiments, wherein a third section, radially even closer to the central longitudinal axis, as compared to the second section, may also have a greater cross-sectional width as compared to the second section.

In a highly preferred embodiment of the invention, the groove-like cut-outs may be configured radially opposite to the ribs. Preferably, this configuration is achieved by an embodiment of the implant, which comprises an uneven number of longitudinal ribs and longitudinal groove-like cut-outs, and wherein the number of longitudinal ribs is the same as the number of longitudinal groove-like cut-outs. The number of longitudinal ribs and longitudinal groove-like cut-outs may each be at least three, at least five and/or at least seven. Preferably, the number of said grooves and said cut-outs is at least three.

The implant according to the present invention provides significant advantages over the prior art. The front part comprising ribs as described alternating with groove-like cut-outs opening towards the front end of the implant has a small cross-sectional area. Thus, the volume of spongiosa, which is displaced when the implant is driven into the bone, is minimized. Due to the small cross-sectional area, the force required to drive the implant into the bone is reduced in comparison to prior art devices. Especially when the implant is implanted into a bone, with a groove-like cut-out facing into the direction from which most mechanical force is exerted onto the implant, an optimized transfer of mechanical force from the bone on the implant is achieved.

Surprisingly, the minimized spongiosa displacement is combined with a high stability, especially a high bending stability of the implant. Especially substantially "T"-formed ribs provide a high bending stability, while requiring few material. Furthermore, although the above-described design of the front part of the implant reduces spongiosa displacement upon insertion of the device, the ribbed structure of the front section of the implant advantageously provides a high rotational stability of the implant in the bone.

Furthermore, the implant according to the present invention advantageously provides a maximal surface area in radial projection and therefore minimizes the pressure exerted from the bone structure onto the implant. Consequently, mechanical stress on the spongiosa and the implant is reduced. The structure of the front section comprising ribs and cut-outs according to the present invention furthermore advantageously increases the total contact area of the implant that engages with the bone. Thus, the friction between the implant and the bone structure is increased and thus backing out of the implant from the bone may advantageously be prevented.

The structure of the implant as described herein, especially the structure of the front section, may advantageously prevent the backing out of the implant from the bone concomitantly facilitates the insertion into the bone without the need of a thread on the outer surface of the implant. Thus, in a preferred embodiment the front section, and/or the intermediate section are non-threaded, especially the outer surface of the front section, and/or the intermediate section and/or the shaft section, or the entire implant may be non-threaded.

As disclosed above, the implant may be straight axially inserted without rotation into a variety of different bones. Preferably, the implant may be implanted into the femur, preferably with the front part of the implant implanted into the femur head and/or neck. Preferably, one groove-like cut-out is directed into a cranial direction upon implantation.

Preferably, the implant according to the present invention is used for treating bone fractures, preferably fractures of long bones, more preferably fractures of the humerus or the femur, most preferably proximal femur fractures.

In a preferred embodiment of the present invention, the implant according to the invention is used for treating bone fractures in assembly with a second medical device suitable for treating bone fractures. Preferably, the second medical device may be a bone plate or a nail, most preferably and intramedullary nail. Thus, one aspect of the present invention relates to an assembly comprising the implant according to the present invention assembled into an intramedullary nail.

In a highly preferred embodiment according to the present invention, the implant according to the present invention is inserted through a transversal bore of an intramedullary nail, which is preferably implanted into the femur, preferably to treat a femur fracture. In this embodiment, the shaft section of the implant according to the present invention engages with the intramedullary nail and the front section of the implant is inserted into the femur head and/or neck, most preferably with one groove-like cut-out directed into a cranial direction.

When an assembly of the implant according to the present invention inserted into an intramedullary nail, as described afore, is used for treating femur fractures, the highest force is applied on the front section of the implant from a cranial direction. Thus, inserting the implant with a groove-like cut-out directed into a cranial direction advantageously preserves spongiosa material in the area that bears the highest mechanical forces and thus advantageously enables an optimized force distribution, bone preservation and consequently may prevent backing out of the implant from the bone.

In a further preferred embodiment of the present invention, the ribs may be configured with an off-set from 100° to 140°, preferably from 110° to 130°, more preferably from 115° to 125°, most preferably of 120°. When the ribs are off-set, for example, by 120°, the angle between the main axis in a radial direction of the two neighboring ribs is 120°.

In a further preferred embodiment of the invention, the protruding ends of the ribs, thus the end of the ribs opposite to the longitudinal axis of the implant, comprise convex outer surfaces directed in a radial direction. Preferably, in a cross-section profile of the front section, the convex outer surfaces of the ribs all substantially align with a circle, having the longitudinal axis of the implant in its center. The outer surfaces of the ribs preferentially extend longitudinally in parallel to the central longitudinal axis of the implant.

In a further embodiment, the ribs comprise concave surfaces directed towards the longitudinal groove-like cut-outs. Thus, the concave surfaces of the ribs at least partially define the shape of the groove-like cut-outs. The design of the ribs constituted by convex and concave surfaces advantageously enables the ribs to bear a high mechanical force at a low usage of material.

In a preferred embodiment, the groove-like cut-outs may be wider than the ribs. In an embodiment wherein the groove-like cut-outs are wider than the ribs, the distance between the longitudinal extending edges of the same rib is smaller than the distance between the longitudinal extending edges of two neighboring ribs that constitute a groove-like cut-out.

In a further preferred embodiment of the implant according to the present invention, the edges at the front end of the implant may be configured as cutting edges, preferably the cutting edges may be configured chisel-like. Thus, the edges at the front end of the implant may preferably be configured to minimize the surface area of the implant directed into an outward axial direction. The cutting edges at the front end of the implant advantageously decrease the forces required for insertion of the implant into the bone, especially the spongiosa. Furthermore, the cutting edges reduce bone destruction during insertion of the implant. Preferably, the front end of the convex surfaces of the ribs comprises cut-outs configured to decrease the width of the front edges of the ribs. Furthermore, the groove-like cut-outs may be chamfered towards the front edge of the implant.

In a preferred embodiment of the implant according to the present invention, the groove-like cut-outs comprised in the front section may taper off towards the shaft of the implant. This tapering off results in a decreased depth of the groove-like cut-outs from the front end to the end of the groove-like cut-outs directed to the shaft. When the groove-like cut-outs taper off towards the shaft, the distance between the bottom of the groove-like cut-outs and the central longitudinal axis in the longitudinal cross-section of the implant increases.

The cut-outs may taper off linearly in at least one section of the cut-outs. The cut-outs may also taper off non-linearly, preferably concavely, in at least one section of the cut-outs. A non-linear tapering of the cut-outs corresponds to a curved shape of the bottom of the cut-out in the longitudinal cross-section of the implant. Most preferably, at least the shaft-directed end section of the cut-outs may taper off concavely towards the shaft. The sections tapering off linearly and/or sections tapering off concavely may taper off with a different pitch. The groove-like cut-outs may also comprise several sections that taper off linearly with a different pitch and/or several sections that taper off concavely with a different pitch. The groove-like cut-outs may also comprise at least one section that is untapered. The cut-outs may also comprise sections that taper off linearly and/or sections that taper off concavely and/or sections that are untapered.

Preferably, in a further preferred embodiment, the groove-like cut-outs may taper off towards the shaft over the entire length of the groove-like cut-outs. Within this embodiment, the cut-outs may comprise at least one section that tapers off linearly and at least one section that tapers off non-linearly, preferably concavely.

In a further preferred embodiment of the implant according to the present invention, the groove-like cut-outs may comprise at least one middle section between a front section of the cut-outs and a shaft-directed end section, wherein the middle section tapers off linearly towards the shaft and the shaft-directed end section tapers off concavely towards the shaft. Within this embodiment, the middle section and the shaft-directed end section may comprise different sections that taper off with a different pitch.

In another preferred embodiment, the groove-like cut-outs comprise an untapered middle section between a front section of the cut-outs and a shaft-directed tapered end section, wherein the bottom of the cut-outs in said middle section is parallelly aligned with the longitudinal axis of the implant in the longitudinal cross-section, and the shaft-directed end section tapers off towards the shaft.

When the implant is driven into the bone during the implantation procedure, the tapered sections of the cut-outs advantageously cause a densification of the spongiosa, which leads to an improved stability of the implant in the bone.

According to a further embodiment, the implant comprises a cannulation along the longitudinal axis of the implant, which opens to the front and the rear end of the implant. The cannulation may be sized to permit insertion of a guide wire to aid the alignment of the implant during the implantation procedure, as commonly known in the art. Additionally, the cannulation provides a possibility to insert / inject a liquid or viscous material through the implant. The cannulation may have the same diameter over the whole length of the implant or may have different sections along the longitudinal axis of the implant with different diameters.

In a further embodiment of the present invention, the bottom of the groove-like cut-outs may comprise at least one opening, preferably several openings, which connect the void of the cut-outs with the cannulation. After insertion of the implant into the bone, bone cement or a similar composition that promotes the fixation of the implant in the bone may be injected into the cannulation and thereby provided to the bone area surrounding the implant through the aforementioned openings. Thus, the openings may advantageously enhance the fixation of the implant in the bone.

In a specific further embodiment of the above described embodiment comprising openings connecting the void of the cut-outs with the cannulation, a tube is provided within the cannulation. Furthermore, two strips extending in a longitudinal direction are provided between the outer wall of the tube and the opposite wall of the cannulation. The strips are configured to bend outward through the aforesaid openings upon exertion of a mechanical force on the strips, wherein said force is directed from the rear end of the implant towards the front end of the implant. The implant may furthermore comprise means for fixing the strips in the bended configuration. The bended strips which protrude into the spongiosa may advantageously further restrict a rotational or lateral movement of the implant in the bone. A respective design feature is for example discloses in U.S. 2008/0262497.

As disclosed above, the implant according to the present invention may be inserted through a transversal bore of a second bone implant, preferably an intramedullary nail. The diameter of the shaft section of the implant according to the present invention may preferably correspond to the diameter of the transversal bore of the second bone implant. More preferably, the diameter of the shaft section of the implant according to the present invention is smaller than the diameter of the transversal bore. In its final position, the shaft section of the implant according to the present invention may be engaged with the second bone implant. To restrict rotation of the implant according to the present invention in relation to the second implant, the shaft section of the implant according to the present invention preferably may comprise at least one element configured to restrict said rotation. Preferably, the at least one element for restricting rotation is at least one notch extending in axial direction comprised in the surface of the shaft section. The notch may preferably be configured to receive a locking element such as a screw, a pin or a similar longitudinal element comprised in the second bone implant. Upon insertion of the locking element into the notch of the implant according to the present invention, rotation of the implant according to the present invention in relation to the second implant is prevented.

In another preferred embodiment, the at least one notch is facing into the same direction as one of the groove-like cut-outs of the front section of the implant. Thus, when an implant according to the present invention is inserted into an intramedullary nail implanted into the femur, one groove-like cut-out is advantageously directed into cranial direction, as described above, while the locking element can advantageously be advanced into the notch from the proximal end of the intramedullary nail. The implant according to the present invention may comprises more than one notches directed into different directions.

In a further preferred embodiment of the implant, the diameter of the front section of the implant may be smaller than the diameter of the shaft section of the implant. Within this embodiment, the diameter of the front section may be defined by the radially protruding ribs. A smaller diameter of the front section advantageously facilitates the insertion of the implant into a transversal bore of a second implant, such as an intramedullary nail.

In addition to a front section and a shaft section, the implant may preferably furthermore comprise an intermediate section between the front section and the shaft section with a diameter tapering from the diameter of the front section to the diameter of the shaft section. The intermediate section may taper off linearly or non-linearly. The intermediate section may also comprise different sections that taper off with a different pitch.

The rear end of the shaft section of the implant according to the present invention may be configured and dimensioned for attachment to an insertion device that facilitates the handling of the implant during the implantation procedure. The insertion device may for example be an insertion handle or driving cap. The rear end of the shaft section of the implant may have a non-symmetrical shape, for example at least one recess, so that the insertion instrument can only be attached in one orientation to the implant. Furthermore, the non-symmetrical shape of the rear end of the implant prevents a rotation of the implant relative to the insertion instrument after attachment of the instrument. Furthermore, the rear end of the shaft may be beveled. The beveled end of the shaft may prevent soft tissue irritation by parts of the rear end of the implant protruding from the bone.

In a further embodiment of the invention, the rear end of the cannulation comprises at least one thread. The at least one thread might be configured for attaching an insertion or extraction instrument. Preferably, the rear end of the cannulation comprises two threads, wherein the thread directed to the front of the implant has a smaller diameter and the thread directed to the rear end of the implant has a larger diameter. Preferably, the two threads run in opposite directions. Preferably, the thread directed to the front end of the device is a left-handed thread, which may be configured to engage with an extraction instrument. Preferably, the thread directed to the rear end of the implant is a right-handed thread configured to engage with an insertion instrument. The at least one thread in the rear end of the implant is configured in a bore or cannulation. Thus, the at least one thread is preferably not configured on the outer surface of the implant.

In a further aspect, the present invention relates to the use of the implant according to the present invention as described above, for treating bone fractures, preferably fractures of a long bone, more preferably a fracture of the humerus or the femur, most preferably fractures of the femur. Accordingly, the present invention also relates to the use of the implant according to the present invention for implantation into a bone, preferably into a long bone, more preferably into the humerus or the femur, most preferably into the femur. In another preferred embodiment, the implant according to the present invention is used for treating bone fractures and/or implantation into a bone, in assembly with a second bone implant, preferably an intramedullary nail. Preferably, the implant according to the present invention is used for implantation and/or treating fractures, wherein one groove-like cut-out of the implant is directed into cranial direction upon implantation into a bone, preferably the femur. Accordingly, the present invention also relates to implant as described herein for use in a method comprising the implantation of said implant into the femur, wherein one groove-like cut-out is directed into cranial direction upon implantation.

Also described herein is a method of surgery or method of treating bone fractures, wherein said method comprises at least the step of implanting an implant according to the present invention into a bone. The method may furthermore comprise a step of inserting the implant according to the present invention into another bone implant.

In the following, embodiments of the implant according to the present invention are explained in reference to the attached drawings, wherein
Fig. 1 shows an implant according to the present invention in lateral view partially from the front;
Fig. 2 shows an implant according to the present invention in a lateral view, partially from the rear;
Fig. 3 shows the front section of the implant according to the invention in a front/side view;
Fig. 4 shows an implant according to the present invention in a front view;
Fig. 5 shows a cross-section through the front part of the implant according to the invention comprising ribs and cut-outs;
Fig. 6 shows a longitudinal section through the front part of an implant according to the invention, comprising a groove-like cut-out with a linearly tapering section;
Fig. 7 shows a longitudinal section through the front part of an implant according to the invention, comprising a groove-like cut-out with an untapered section; and
Fig. 8 shows a longitudinal section through an entire implant according to the present invention, wherein the groove-like cut-outs comprise openings.

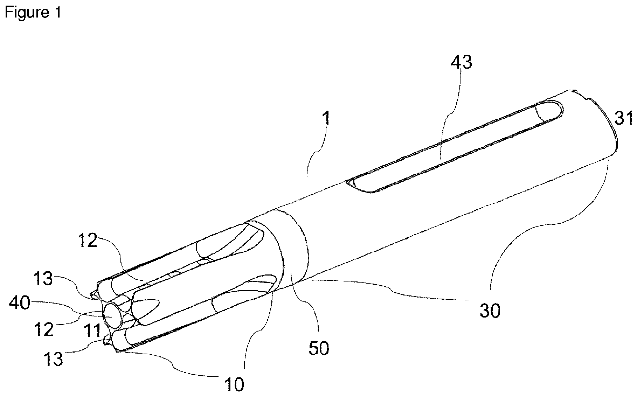

In Figs. 1 to 8, a longitudinal bone implant 1 with a substantially circular cross-section profile, comprising a front section 10 having a front end 11 and a shaft section 30 having a rear end 31, wherein the front section 10 comprises at least three longitudinal groove-like cut-outs 12 extending in the axial direction of the front section 10 and opening towards the front end 11 of the implant, circumferentially alternating with at least three longitudinal protruding ribs 13 extending in an axial direction is shown. The embodiments exemplified in the figures all have a configuration wherein the groove-like cut-outs 12 are configured radially opposite to the ribs 13.

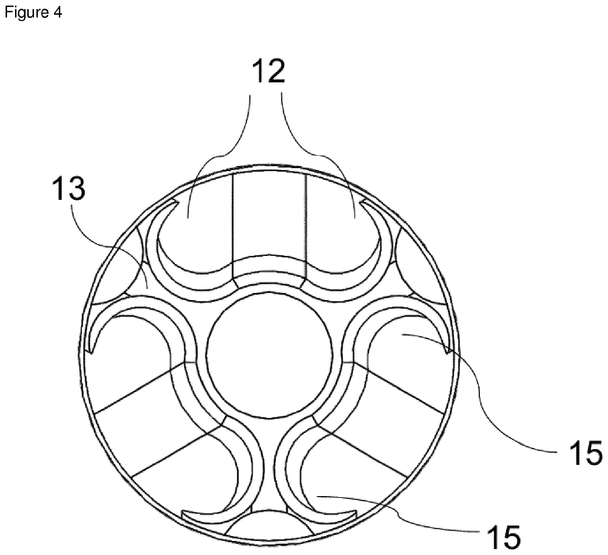

The substantially circular cross-sectional profile of the implant corresponds to the view along the longitudinal axis of the implant, which is for example depicted in Fig. 4.

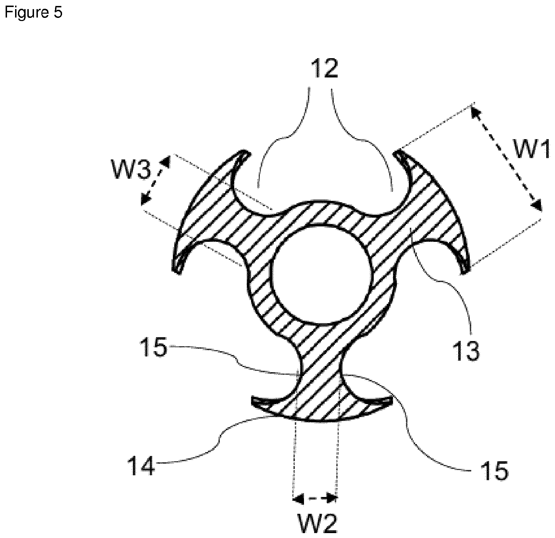

As shown in Fig. 5, the ribs 13 in the front section 10 of the implant may have an increased cross-sectional width (W1) in a section radially more distant to the central longitudinal axis of the implant, as compared to the width (W2) in a section radially closer to the central longitudinal axis of the implant. Furthermore, the ribs 13 may comprise a third section, radially even closer to the central longitudinal axis, as compared to the second section, which may have a greater cross-sectional width (W3) as compared to the second section. The embodiment shown in Fig. 5 encompasses ribs which are cross-sectionally substantially T-formed, wherein the base of the "T" is directed towards the longitudinal axis 40 of the implant.

In the embodiments shown in Figs. 1 to 8, the protruding ends of the ribs, thus the ends of the ribs opposite to the longitudinal axis of the implant, comprise convex outer surfaces 14 directed in the radial direction. The convex outer surfaces of the ribs all substantially align with a circle having the longitudinal axis 40 of the implant in its center, which is, for example, schematically shown in Figs. 4 and 5. Furthermore, the ribs comprise concave surfaces 15 directed towards the longitudinal groove-like cut-outs.

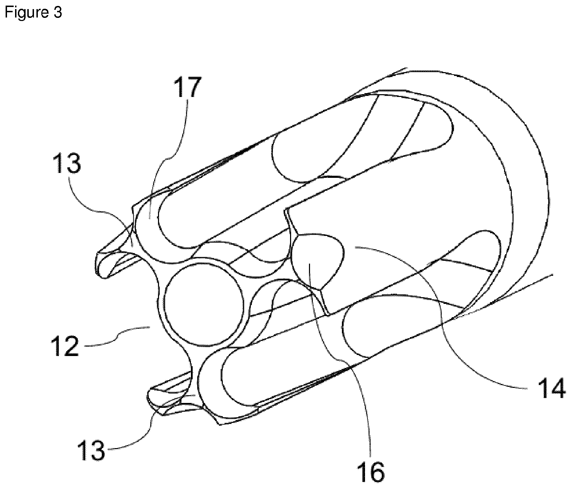

In a preferred embodiment of the implant according to the invention, the edges at the front end 10 of the implant may preferably be configured to minimize the surface area of the implant directed into outward axial direction. Therefore, as shown in Fig. 3, the front end of the convex surfaces of the ribs 13 comprise cut-outs 16 configured to decrease the width of the front edges of the ribs 13. Furthermore, the groove-like cut-outs 12 may comprise chamfered sections 17 towards the front edge of the implant.

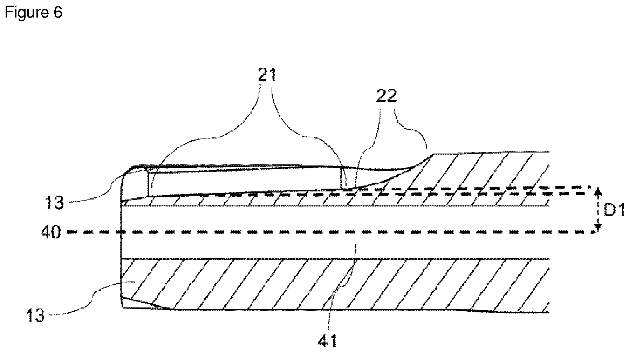

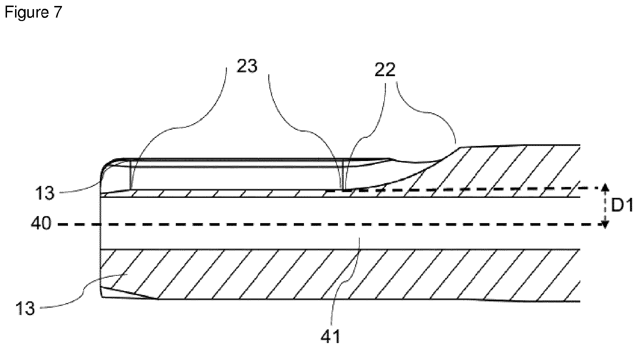

In some embodiments of the invention, the groove-like cut-outs 12 comprised in the front section 10 may taper off towards the shaft section 30 of the implant, as, for example, shown in Figs. 6 and 7. When the groove-like cut-outs 12 taper off towards the shaft section, the distance D1 in the longitudinal cross-section of the implant between the bottom of the groove-like cut-outs and the central longitudinal axis 40 increases. The implant shown in Fig. 6 exhibits cut-outs with a section 21 that tapers off linearly and a section 22 that tapers off concavely. The implant shown in Fig. 7 exhibits cut-outs with an untapered section 23 and a section 22 that tapers off concavely.

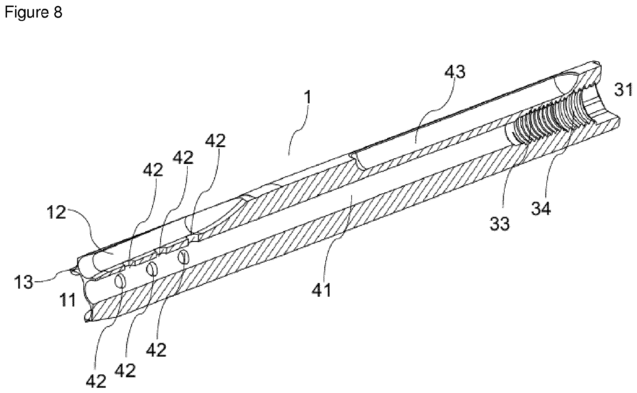

In the embodiments shown in Figs. 1 to 8, the implant according to the invention comprises a cannulation 41 along the longitudinal axis 40 of the implant opening to the front 11 and the rear end 31 of the implant. In the embodiment of the implant shown in Fig. 8, the bottom of the groove-like cut-outs 12 comprises openings 42 which connect the void of the cut-outs with the cannulation 41.

As shown in Figs. 1, 2 and 8, the shaft section 30 of the implant according to the present invention may comprise a notch 43 in the surface of the shaft section 30 for restricting rotation of the implant in relation to a second implant. In the depicted embodiments, the notch 43 is phasing into the same direction as one of the groove-like cut-outs 12 of the front section 10 of the implant.

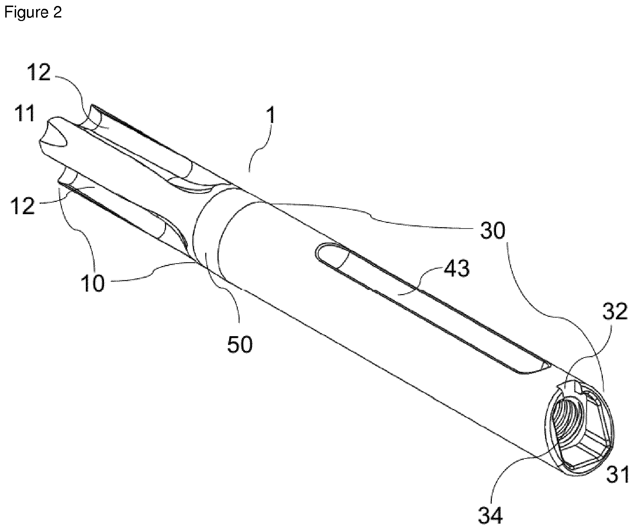

As shown in Figs. 1, 2 and 8, the implant may preferably furthermore comprise an intermediate section 50 between the front section 10 and the shaft section 30 with a diameter tapering from the diameter of the front section 10 to the diameter of the shaft section 30. As shown, for example, in Fig. 2, the rear end 31 of the shaft section 30 of the implant may be configured and dimensioned for attachment of an insertion device that facilitates the handling of the implant during the implantation procedure. For example, the rear end 31 of the shaft section may have a recess 32. The embodiments shown in Figs. 2 and 8 furthermore comprise two threads 33, 34, wherein the thread 33 directed to the front end of the implant has a smaller diameter when the thread 34 directed to the rear end 31 of the implant. The two threads may run in opposite directions.

The implant according to the present invention, as described above and exemplified in the figures, surprisingly solve the problem underlying the invention and provide significant advantages over the prior art.

The front section of the implant comprising ribs alternating with groove-like cut-outs has a minimal cross-sectional area. Thus, when the front end of the implant is driven into a bone, the volume of a spongiosa which is displaced by the implant is minimized. This minimized spongiosa displacement is advantageously combined with a high stability, especially bending stability, of the ribs in the implant of the present invention. Furthermore, the forces for insertion of the implant into the bone are reduced. Advantageously, the implant can be implanted into a bone with a groove-like cut-out facing into the direction from which the highest mechanical force is exerted onto the implant. Thereby, only little spongiosa is displaced in the direction from which the force is applied, which enables an optimized transfer of the mechanical force from the bone to the implant.

When the implant is used for the treatment of femur fractures, one groove-like cut-out may be directed in the cranial direction, which advantageously preserves spongiosa material in the area that bears the highest mechanical forces, and thus advantageously enables optimized force distribution and bone preservation.

Especially the substantially T-formed ribs provide a high bending stability while requiring only little material. Furthermore, although the above-described design of the front part of the implant reduces spongiosa displacement upon insertion of the device into the bone, the ribbed structure of the front section of the implant advantageously provides a high rotational stability of the implant in the bone.

The implant according to the present invention advantageously provides a maximal support area in radial projection and therefore generally minimizes the pressure exerted from the bone structure onto the implant and thus relieves the spongiosa and the implant. The structure of the front section comprising ribs and cut-outs according to the present invention, furthermore advantageously increases the total contact area of the implant that engages with the bone. Thus, the friction between the implant and the bone structure is increased and consequently the backing out of the implant from the bone is advantageously prevented. In addition, the tapered sections of the cut-outs advantageously cause a densification of the spongiosa, which leads to an improved stability of the implant in the bone.

Surprisingly, when comparing the stability of a bone implant according to the present invention, comprising a front section with groove like cut-outs, with state of the art helical blade implants, it was found that implant according to the invention exhibited a higher stability in an artificial bone model. While exertion of high forces on implants according to the invention inserted into a cellular rigid polyurethane foam only induced a slight migration, the same force induced a cutting-out of the helical blades from the test material.

Furthermore, the cutting edges at the front of the implant advantageously decrease the forces required for insertion of the implant into the bone.

## Claims

1. A longitudinal bone implant (1) with a substantially circular cross-sectional profile, comprising a front section (10) having a front end a (11) and a shaft section (30) having a rear end (31), wherein the front section comprises, at least three longitudinal groove-like cut-outs (12) extending in axial direction of the front section and opening towards the front end of the implant, circumferentially alternating with at least three longitudinal, radially protruding ribs (13) extending in axial direction, wherein the ribs have an increased cross-sectional width in a section radially more distant to the central longitudinal axis of the implant as compared to the width in a section radially closer to the central longitudinal axis of the implant, **characterised in that** the front section is a non-threaded front section.

2. The implant of claim 1, wherein the groove-like cut-outs are configured radially opposite to the ribs.

3. The implant of any of the preceding claims, wherein the ribs are cross-sectional substantially T-formed.

4. The implant of any of the preceding claims, wherein the edges at the front end of the implant are configured as cutting edges.

5. The implant of any of the preceding claims, wherein the protruding end of the ribs comprises convex outer surfaces (14) directed in radial direction and/or the ribs comprise concave surfaces (15) directed towards the longitudinal groove-like cut-outs.

6. The implant of claim 5, wherein the front end of the convex surface of the ribs comprises cut-outs configured to decrease the width of the front edges of the ribs and/or wherein the groove-like cut-outs are chamfered towards the front edge of the implant.

7. The implant of any of the preceding claims, wherein the groove-like cut-outs taper off towards the shaft of the implant.

8. The implant of any of the preceding claims, wherein the groove-like cut-outs taper off towards the shaft over the entire length of the groove-like cut-outs.

9. The implant of any of the preceding claims, wherein the groove-like cut-outs comprises a middle section between a front section of the cut-out and a shaft directed end section, wherein the middle section tapers-off linearly towards the shaft and the shaft directed end section tapers-off concavely towards the shaft.

10. The implant of any of claims 1 to 7, wherein the groove-like cut-outs comprises an untapered middle section between a front section of the cut-out and a shaft directed tapered section end section, wherein the bottom of the cut-out in said middle section is parallelly aligned with the longitudinal axis of the implant, and the shaft directed end section tapers-off towards the shaft

11. The implant of any of the preceding claims, wherein the implant comprises a cannulation (41) along the longitudinal axis of the implant opening to the front and the rear end of the implant.

12. The implant of any of the preceding claims, wherein the surface of the shaft comprises at least one notch (43) extending in axial direction and preferably facing into the same direction as one of the cut-out.

13. The implant of any of the preceding claims, wherein the diameter of the front section of the implant, as defined by the radially protruding ribs, is smaller than the diameter of the shaft of the implant.

14. The implant of any of the preceding claims for use in a method comprising the implantation of said implant into the femur, wherein one groove-like cut-out is directed into cranial direction upon implantation.

15. An assembly comprising the implant of any of the preceding claims assembled into an intramedullary nail.

## Patentansprüche

1. Längliches Knochenimplantat (1)
mit einem im wesentlichen kreisförmigen Querschnittsprofil,
umfassend einen vorderen Abschnitt (10) mit einem vorderen Ende (11) und einen Schaftabschnitt (30) mit einem hinteren Ende (31),
wobei der vordere Abschnitt wenigstens drei längsverlaufende rillenartige Ausschnitte (12) umfasst, die sich von dem vorderen Abschnitt in axialer Richtung erstrecken und sich zu dem vorderen Ende des Implantats hin öffnen,
welche sich umlaufend mit wenigstens drei längsverlaufenden, radial hervorstehenden Rippen (13) abwechseln, die sich in axialer Richtung erstrecken,
wobei die Rippen eine vergrößerte Querschnittsbreite in einem Abschnitt aufweisen, der radial weiter von der zentralen Längsachse des Implantats entfernt ist, im Vergleich zur Breite in einem Abschnitt, der radial näher an der zentralen Längsachse des Implantats liegt,
**dadurch gekennzeichnet, dass**
der vordere Abschnitt ein gewindeloser vorderer Abschnitt ist.

2. Implantat nach Anspruch 1, wobei die rillenartigen Ausschnitte radial gegenüber den Rippen ausgestaltet sind.

3. Implantat nach einem der vorhergehenden Ansprüche, wobei die Rippen einen im wesentlichen T-förmigen Querschnitt aufweisen.

4. Implantat nach einem der vorhergehenden Ansprüche, wobei die Kanten am vorderen Ende des Implantats als Schneidkanten ausgestaltet sind.

5. Implantat nach einem der vorhergehenden Ansprüche, wobei das hervorstehende Ende der Rippen konvexe äußere Oberflächen (14) umfasst, die in radialer Richtung gerichtet sind, und/oder die Rippen konkave Oberflächen (15) umfassen, die auf die länglichen rillenförmigen Ausschnitte gerichtet sind.

6. Implantat nach Anspruch 5, wobei das vordere Ende der konvexen Oberfläche der Rippen Ausschnitte aufweist, die ausgestaltet sind, um die Breite der Vorderkanten der Rippen zu verringern, und/oder wobei die rillenartigen Ausschnitte zur Vorderkante des Implantats hin abgeschrägt sind.

7. Implantat nach einem der vorhergehenden Ansprüche, wobei sich die rillenartigen Ausschnitte zum Schaft des Implantats hin verjüngen.

8. Implantat nach einem der vorhergehenden Ansprüche, wobei sich die rillenartigen Ausschnitte über die gesamte Länge der rillenartigen Ausschnitte zum Schaft hin verjüngen.

9. Implantat nach einem der vorhergehenden Ansprüche, wobei die rillenartigen Ausschnitte einen Mittelabschnitt zwischen einem vorderen Abschnitt des Ausschnitts und einem auf den Schaft gerichteten Endabschnitt umfassen, wobei sich der Mittelabschnitt linear zum Schaft hin verjüngt und sich der auf den Schaft gerichtete Endabschnitt konkav zum Schaft hin verjüngt.

10. Implantat nach einem der Ansprüche 1 bis 7, wobei die rillenartigen Ausschnitte einen nicht verjüngten Mittelabschnitt zwischen einem vorderen Abschnitt des Ausschnitts und einem auf den Schaft gerichteten sich verjüngenden Abschnitt Endabschnitt umfassen, wobei der Boden des Ausschnitts im Mittelabschnitt parallel zur Längsachse des Implantats ausgerichtet ist, und wobei der auf den Schaft gerichtete Endabschnitt sich zum Schaft hin verjüngt.

11. Implantat nach einem der vorhergehenden Ansprüche, wobei das Implantat entlang der Längsachse des Implantats eine Durchbohrung (41) umfasst, die sich zum vorderen und hinteren Ende des Implantats öffnet.

12. Implantat nach einem der vorhergehenden Ansprüche, wobei die Oberfläche des Schafts mindestens eine Kerbe (43) aufweist, die sich in axialer Richtung erstreckt und vorzugsweise in die gleiche Richtung wie einer der Ausschnitte zeigt.

13. Implantat nach einem der vorhergehenden Ansprüche, wobei der Durchmesser des vorderen Abschnitts des Implantats, wie er durch die radial hervorstehenden Rippen definiert ist, kleiner als der Durchmesser des Schafts des Implantats ist.

14. Implantat nach einem der vorhergehenden Ansprüche zur Verwendung in einem Verfahren, welches die Implantation des Implantats in das Femur umfasst, wobei ein rillenartiger Ausschnitt bei der Implantation in kraniale Richtung gerichtet ist.

15. Baugruppe, umfassend das Implantat eines der vorhergehenden Ansprüche, das in einen Marknagel montiert ist.

## Revendications

1. Implant osseux longitudinal (1), avec un profil en section transversale sensiblement circulaire, comprenant une section avant (10) ayant une extrémité avant (11) et une section de tige (30) ayant une extrémité arrière (31), la section avant comprenant au moins trois découpes longitudinales de type rainure (12) s'étendant dans la direction axiale de la section avant et ouvrant vers l'extrémité avant de l'implant, en alternance sur la circonférence avec au moins trois nervures longitudinales saillant radialement (13), s'étendant dans la direction axiale, les nervures ayant une largeur en section transversale accrue dans une section radialement plus éloignée de l'axe longitudinal central de l'implant par comparaison avec la largeur dans une section radialement plus proche de l'axe longitudinal central de l'implant, **caractérisé en ce que** la section avant est une section avant non filetée.

2. Implant selon la revendication 1, dans lequel les découpes de type rainure sont configurées radialement à l'opposé des nervures.

3. Implant selon l'une quelconque des revendications précédentes, dans lequel les nervures sont sensiblement en forme de T en section transversale.

4. Implant selon l'une quelconque des revendications précédentes, dans lequel les bords au niveau de l'extrémité avant de l'implant sont configurés sous forme de bords de coupe.

5. Implant selon l'une quelconque des revendications précédentes, dans lequel l'extrémité saillante des nervures comprend des surfaces convexes extérieures (14) orientées dans la direction radiale et/ou les nervures comprennent des surfaces concaves (15) orientées vers les découpes longitudinales de type rainure.

6. Implant selon la revendication 5, dans lequel l'extrémité avant de la surface convexe des nervures comprend des découpes configurées pour diminuer la largeur des bords avant des nervures et/ou dans lequel les découpes de type rainure sont chanfreinées vers le bord avant de l'implant.

7. Implant selon l'une quelconque des revendications précédentes, dans lequel les découpes de type rainure sont effilées vers la tige de l'implant.

8. Implant selon l'une quelconque des revendications précédentes, dans lequel les découpes de type rainure sont effilées vers la tige sur toute la longueur des découpes de type rainure.

9. Implant selon l'une quelconque des revendications précédentes, dans lequel les découpes de type rainure comprennent une section centrale entre une section avant de la découpe et une section d'extrémité dirigée vers la tige, la section centrale étant effilée linéairement vers la tige et la section d'extrémité dirigée vers la tige étant effilée sous forme concave vers la tige.

10. Implant selon l'une quelconque des revendications 1 à 7, dans lequel les découpes de type rainure comprennent une section centrale non effilée entre une section avant de la découpe et une section d'extrémité de la section effilée dirigée vers la tige, la base de la découpe dans ladite section centrale étant alignée parallèlement avec l'axe longitudinal de l'implant, et la section d'extrémité dirigée vers la tige étant effilée vers la tige.

11. Implant selon l'une quelconque des revendications précédentes, l'implant comprenant une canulation (41) le long de l'axe longitudinal de l'implant, ouvrant vers les extrémités avant et arrière de l'implant.

12. Implant selon l'une quelconque des revendications précédentes, dans lequel la surface de la tige comprend au moins une encoche (43) s'étendant dans la direction axiale et tournée de préférence dans la même direction que l'une des découpes.

13. Implant selon l'une quelconque des revendications précédentes, dans lequel le diamètre de la section avant de l'implant, tel que défini par les nervures saillant radialement, est inférieur au diamètre de la tige de l'implant.

14. Implant selon l'une quelconque des revendications précédentes, pour l'utilisation dans un procédé comprenant l'implantation dudit implant dans le fémur, une découpe de type rainure étant dirigée dans la direction crâniale lors de l'implantation.

15. Ensemble comprenant l'implant selon l'une quelconque des revendications précédentes, assemblé dans un clou intramédullaire.
